# FASCICULE DE BREVET EUROPEEN

(11) **EP 2 943 588 B1**
(45) Date de publication et mention de la délivrance du brevet: **05.07.2017**
(21) Numéro de dépôt: 14703127.2
(22) Date de dépôt: 10.01.2014
(51) Int. Cl.: C12Q 1/68

(54) **PROCEDE ET KIT POUR ETABLIR IN VITRO UN PRONOSTIC DE SEVERITE CHEZ UN PATIENT EN CHOC SEPTIQUE**
VERFAHREN UND KIT ZUR IN-VITRO-ERSTELLUNG EINER PROGNOSE DER SCHWERE EINES SEPTISCHEN SCHOCKS BEI EINEM PATIENTEN
METHOD AND KIT FOR ESTABLISHING, IN VITRO, A PROGNOSIS OF SEVERITY IN A SEPTIC SHOCK PATIENT

(30) Priorité: 11.01.2013 FR 1350252
(43) Date de publication de la demande: 18.11.2015
(73) Titulaire: Biomérieux, 69280 Marcy l'Étoile (FR); Hospices Civils de Lyon, 69002 Lyon (FR)
(72) Inventeur: CAZALIS, Marie-Angélique, 69120 Vaulx en Velin (FR); TOURNOUD, Maud, 38000 Grenoble (FR); VENET, Fabienne, 69008 Lyon (FR); LEPAPE, Alain, 69230 Saint Genis Laval (FR); MONNERET, Guillaume, 69003 Lyon (FR)
(86) Numéro de dépôt international: PCT/FR2014/050039
(87) Numéro de publication internationale: WO 2014/108646

(56) Documents cités:
- WO-A2-2004/087949
- WO-A2-2008/143890
- US-A1- 2004 241 167
- HECTOR R. WONG ET AL: "Genomic expression profiling across the pediatric systemic inflammatory response syndrome, sepsis, and septic shock spectrum*", CRITICAL CARE MEDICINE, vol. 37, no. 5, 1 mai 2009 (2009-05-01), pages 1558-1566, XP055076786, ISSN: 0090-3493, DOI: 10.1097/CCM.0b013e31819fcc08
- LEPIN E J M ET AL: "FUNCTIONAL CHARACTERIZATION OF HLA-F AND BINDING OF HLA-F TETRAMERSTO ILT2 AND ILT4 RECEPTORS", EUROPEAN JOURNAL OF IMMUNOLOGY, WILEY - V C H VERLAG GMBH & CO. KGAA, DE, vol. 30, no. 12, 1 janvier 2000 (2000-01-01), pages 3552-3561, XP001024681, ISSN: 0014-2980, DOI: 10.1002/1521-4141(200012)30:12<3552::AID-I MMU3552>3.0.CO;2-L
- HECTOR R WONG: "Clinical review: Sepsis and septic shock - the potential of gene arrays", CRITICAL CARE, vol. 16, 1 janvier 2012 (2012-01-01), pages 204-211, XP055076787,
- THOMAS SHANLEY ET AL: "Genome-Level Longitudinal Expression of Signaling Pathways and Gene Networks in Pediatric Septic Shock", MOLECULAR MEDICINE, vol. 13, no. 9-10, 1 janvier 2007 (2007-01-01), pages 495-508, XP055076790, ISSN: 1076-1551, DOI: 10.2119/2007-00065.Shanley

### SEQUENCE LISTING

<110> bioMérieux Hospices Civils de Lyon
<120> Procédé et kit pour établir in vitro un pronostic de sévérité chez un patient en choc septique
<130> SEPTICHOC PCT
<150> FR1350252
   <151> 2013-01-11
<160> 6
<170> PatentIn version 3.5
<210> 1
   <211> 20
   <212> DNA
   <213> Artificial Sequence
<220>
   <223> LILRB2 PRIMER 1
<400> 1
   ccagagccca cagacagagg 20
<210> 2
   <211> 21
   <212> DNA
   <213> Artificial Sequence
<220>
   <223> LILRB2 PRIMER 2
<400> 2
   tgtccttcac ggcagcatag a 21
<210> 3
   <211> 20
   <212> DNA
   <213> Artificial Sequence
<220>
   <223> LILRB2 SONDE 3
<400> 3
   gactccacac tcagtagaag 20
<210> 4
   <211> 20
   <212> DNA
   <213> Artificial Sequence
<220>
   <223> HPRT1 PRIMER 4
<400> 4
   ccaaagatgg tcaaggtcgc 20
<210> 5
   <211> 22
   <212> DNA
   <213> Artificial Sequence
<220>
   <223> HPRT1 PRIMER 5
<400> 5
   gacacaaaca tgattcaaat cc 22
<210> 6
   <211> 23
   <212> DNA
   <213> Artificial Sequence
<220>
   <223> HPRT1 SONDE 6
<400> 6
   caagtttgtt gtaggatatg ccc 23

## Description

La présente invention concerne un procédé et un kit pour établir *in vitro* un pronostic de sévérité chez un patient en choc septique.

Les syndromes septiques représentent l'une des premières causes de mortalité dans les services de réanimation. Ils peuvent résulter d'une infection bactérienne, virale, mycosique ou parasitaire. Ils se définissent comme un syndrome de réponse systémique inflammatoire ou SIRS (Systemic Inflammatory Response Syndrome).
- le sepsis est un syndrome de réponse systémique inflammatoire en relation avec une infection,
- un sepsis sévère est un sepsis associé à une hypotension artérielle et/ou hypoperfusion et/ou au dysfonctionnement d'au moins un organe,
- le choc septique est un sepsis sévère associé à une hypotension persistante malgré des remplissages adéquats et des traitements vasopresseurs.

La différence entre SEPSIS, SEPSIS sévère et choc septique réside principalement dans l'importance de la perturbation de toutes les fonctions vitales.

Les syndromes septiques ont longtemps été considérés uniquement comme une réponse inflammatoire non régulée conduisant, en réponse à une dissémination bactérienne initiale, à une défaillance multi-viscérales de l'organisme. Cependant, les échecs des approches thérapeutiques, principalement anti-inflammatoires, expérimentées durant ces dernières années, ainsi que l'incapacité à caractériser la forte hétérogénéité des patients septiques ont contribué à remettre en cause la vision essentiellement pro-inflammatoire de la physiopathologie des syndromes septiques.

Actuellement, la réponse immunitaire au cours du choc septique est plutôt décrite en deux phases successives. Après une brève phase initiale très inflammatoire, responsable de la symptomatologie du choc proprement dit, se met en place un état de dépression immunitaire induit par les mécanismes immunosuppresseurs chargés de contrôler la réponse pro-inflammatoire. Ainsi, cette composante anti-inflammatoire de la réaction à l'infection, initialement perçue comme purement compensatrice, s'est révélée prédominante chez la majorité des malades et s'accompagne d'une immunosuppression et d'une hypo-réactivité cellulaire marquée. De plus, il semble que la mortalité survienne principalement à distance du choc (ou de la défaillance d'organe initiale) dans un contexte où tous les patients présentent des signes biologiques d'immunodépression.

De nombreux marqueurs dans un contexte sepsis ont déjà été décrits dans la littérature scientifique.

En effet, Thomas Shanley et al. [1] réalise une étude en 2007 sur les profils d'expression de voies de signalisation et de réseaux de gènes chez les enfants atteints de choc septique. Cette publication montre une expression différentielle d'un nombre très important de gènes, près de 2000 gènes, chez des patients sains et de patients en choc septiques par rapport à des patients sains. En 2009, la même équipe [2] décrit une étude sur le génome d'enfants gravement malades atteints de SIRS, sepsis ou encore de choc septique. Les profils d'expression ont été déterminés à l'aide d'une puce à ADN. Une analyse statistique montre qu'un très grand nombre de gènes, près de 700, sont régulés différemment dans des échantillons prélevés chez des patients atteints de SIRS, sepsis ou un choc septique par rapport des échantillons de patients sains.

La demande WO2008143890 divulgue l'utilisation de IL-8 en tant que biomarqueur pour le pronostic de survie chez des patients en choc septique, pour adapter le choix des traitements et à des fins de structuration, de conduite ou d'évaluation d'essais cliniques ou de données d'essais cliniques.

En 2012, Hector R. Wang [3] réalise une revue bibliographique qui traite des applications des puces à ADN et le profil d'expression génétique dans le domaine du sepsis. Les différentes applications mises en évident sont la compréhension génomique du sepsis, la découverte de nouveau biomarqueurs, l'identification sous-catégories du choc septique basée sur l'expression des gènes et la découverte de nouvelles cibles et voies.

Enfin dans la demande WO 2004/087949 9 A2 il est décrit un procédé de diagnostic *in vitro* d'un SIRS, un sepsis et/ou d'états de type sepsis. Ce procédé permet d'évaluer l'état de gravité et/ou l'évolution du suivi thérapeutique de sepsis et d'infections graves, notamment d'infections systémiques de type sepsis. Ce document divulgue de nombreux gènes pour leur utilisation en tant que biomarqueurs pour le diagnostic d'un SIRS, un sepsis ou un état de type sepsis.

Certains marqueurs ont ainsi été décrits dans la littérature comme pouvant être une aide à un pronostic de mortalité ou de survie chez des patients en choc septique. Mais dans tous les procédés décrits jusqu'à présent, les échantillons de sang sont prélevés une première fois dans les 24 à 72 premières heures après le début du choc (J1-J3), puis plus tardivement au cours du choc septique. En dépit de leur intérêt, la significativité de ces marqueurs ne se révèle au plus tôt qu'à J1-J3. En d'autres termes, ils ne sont pas suffisamment précoces pour une prise en charge très réactive et la plus rapide possible d'un patient en état de choc. Or, une prise en charge adaptée du patient au plus tôt après le choc est indispensable. Il est donc nécessaire et très important de disposer de marqueurs qui permettent de réduire au maximum cette fenêtre pronostique.

De manière complètement inattendue, les présents inventeurs ont montré que les produits d'expression des gènes *lilrb2* et/ou *lilrb1* permettent d'établir dès les toutes première heures et pas plus tard que dans les 12 premières heures après le début du choc, un pronostic de sévérité ou de survie/ mortalité chez des patients en choc septique. Ces gènes appartiennent à la famille des gènes récepteurs des immunoglobulines qui sont localisés sur le chromosome 19q13.4

Il existe plusieurs variants pour les deux gènes cibles qui sont tous pertinents dans le cadre de l'invention. Ces variants sont répertoriés dans Genbank sous les références et numéros d'accession suivantes :
LILRB2:
« Homo sapiens leukocyte immunoglobulin-like receptor, subfamily B (with TM and ITIM domains), member 2 (LILRB2), transcript »

| | |
|---|---|
| NM_005874.3 | variant 1 Refseq |
| NM_001080978.2 | variant 2 Refseq |

LILRB1
« Homo sapiens leukocyte immunoglobulin-like receptor, subfamily B (with TM and ITIM domains), member 1 (LILRB1), transcript »

| | |
|---|---|
| NM_006669.3 | variant 1 Refseq |
| NM_001081637.1 | variant 2 Refseq |
| NM_001081638.1 | variant 3 Refseq |
| NM_001081639.1 | variant 4 Refseq |

Ainsi, la présente invention a pour objet un procédé pour établir *in vitro* un pronostic de sévérité (de survie ou de risque accru de mortalité) chez un patient en choc septique, comprenant les étapes suivantes:
(i) à partir d'un échantillon biologique provenant dudit patient, on mesure *in vitro* le niveau d'expression du produit d'expression d'au moins un gène choisi parmi les gènes *lilrb2* et *lilrb1*, (ii) on compare le niveau d'expression du produit d'expression dudit au moins un gène avec un niveau d'expression contrôle de bon pronostic de sévérité pour le produit d'expression du même gène, dans lequel un niveau d'expression du produit d'expression dudit au moins un gène en-dessous dudit niveau d'expression contrôle est indicatif d'un mauvais pronostic de sévérité pour le patient.

Dans un mode de réalisation préférée, (i) on mesure le niveau d'expression du produit d'expression du gène *lilrb2,* (ii) on compare le niveau d'expression du produit d'expression du gène *lilrb2* avec le niveau d'expression contrôle de bon pronostic, dans lequel un niveau d'expression du produit d'expression du gène *lilr2* du patient en-dessous de celui du niveau d'expression contrôle est indicatif d'un mauvais pronostic pour le patient.

Dans un autre mode de réalisation, (i) on mesure le niveau d'expression du produit d'expression du gène *lilrb2* et le niveau d'expression du produit d'expression du gène *lilrb1* du patient,(ii) on compare le niveau d'expression du produit d'expression du gène *ilrb2* et du gène *ilrb1* du patient respectivement avec le niveau d'expression contrôle de chaque gène,
dans lequel un niveau d'expression du produit d'expression du gène *lilr2* et du gène *lilrb1* du patient au-dessous respectivement du niveau d'expression contrôle de chaque gène est indicatif d'un mauvais pronostic pour le patient.

L'échantillon biologique est choisi parmi le sang, le plasma, le sérum, la salive, l'urine, le liquide céphalo-rachidien, le liquide pleural et le liquide péritonéal.

Le procédé de l'invention peut aussi comprendre une étape supplémentaire selon laquelle dans l'étape on extrait les cellules mononucléees du sang périphérique (PBMCs) de l'échantillon sanguin et on réalise les étapes subséquentes sur lesdites cellules mononucléees sanguines, comme décrit précédemment.

Le produit d'expression du gène ou des gènes est soit un ARN et de préférence un ARN messager ou encore un ADNc, soit une protéine ou un polypeptide. Quand le produit d'expression est un ARN, son niveau d'expression est déterminé à l'aide d'au moins une sonde d'hybridation spécifique du produit d'expression et de préférence à l'aide d'au moins deux voire trois sondes d'hybridation et en particulier par une amplification enzymatique quantitative. Quand le produit d'expression du ou des gènes est un polypeptide ou une protéine son niveau d'expression est déterminé de préférence par un dosage immunologique quantitatif.

L'invention a aussi pour objet un procédé pour réaliser *in vitro* un suivi d'un patient en choc septique, par mesure de l'évolution du niveau d'expression du produit d'expression d'au moins un gène choisi parmi les gènes *ilrb2* et *ilrb1* chez ledit patient, dans lequel
(i) on mesure le niveau d'expression dudit au moins un gène dans un échantillon du patient prélevé à J1, c'est à dire au plus tard dans les 12 premières heures du choc septique,
(ii) on mesure le niveau d'expression dudit au moins un gène dans un échantillon du patient prélevé à Jx, c'est à dire subséquemment à J1,
dans lequel une augmentation dudit niveau d'expression dudit au moins un gène entre J1 et Jx indique que le patient évolue vers un bon pronostic de sévérité.

Bien entendu, l'étape (ii) peut être réalisée plusieurs fois pour suivre l'évolution du niveau d'expression dudit au moins un gène au cours du temps.

Comme décrit ci-dessus le niveau d'expression du produit d'expression est de préférence mesuré par amplification quantitative et par dosage immunologique quantitatif.

### Définitions

Echantillon biologique signifie le sang, le plasma, le sérum, la salive, l'urine, le liquide céphalo-rachidien, le liquide pleural et le liquide péritonéal.

Echantillon de sang ou échantillon sanguin signifie indifféremment le sang total, le sérum ou le plasma.

On entend par produit d'expression d'un gène l'ARN messager ou un fragment d'ARNm, l'ADNc ou un fragment d'ADNc, un polypeptide ou une protéine ou un fragment de polypeptide ou de protéine.

On entend par réactif spécifique un réactif qui réagit avec un matériel biologique de l'échantillon de sang ou des cellules mononucléées du sang périphérique (PBMCs) afin de mettre en évidence, d'une manière directe ou indirecte l'expression d'un gène cible, qui peut être déterminé par l'analyse des ARNm issus de ce gène, ou par l'analyse de la protéine codée par ce gène.

A titre indicatif, lorsque l'on souhaite déterminer l'expression d'un gène cible par l'analyse de la protéine codée par ce gène, ce réactif spécifique comprend au moins un anticorps spécifique de la protéine codée par ce gène cible.

A titre indicatif, lorsque l'on souhaite déterminer l'expression d'un gène cible par l'analyse des ARNm transcrits à partir de ce gène, ce réactif spécifique comprend au moins une amorce d'amplification spécifique de l'ADN complémentaire de cet ARNm (on parle alors d'amorce d'amplification spécifique d'un gène cible). L'ADN complémentaire d'un ARNm peut être obtenu selon un protocole bien connu de l'homme du métier. Par exemple, on extrait les ARN totaux (comprenant les ARN ribosomaux, les ARN de transfert, les ARNm) de l'échantillon sanguin ou des PBMCs. On réalise ensuite une réaction de transcription inverse à l'aide d'une enzyme transcriptase inverse qui permet d'obtenir, à partir d'un fragment d'ARN, un fragment complémentaire d'ADN (ADNc). La réalisation d'une telle étape est bien connue de l'homme du métier. Lorsque l'on souhaite plus particulièrement obtenir uniquement les ADN complémentaires des ARN messagers, on réalise cette étape enzymatique en présence de fragments nucléotidiques comprenant uniquement des bases thymine (polyT), qui s'hybrident par complémentarité sur la séquence polyA des différents ARNm afin de former un complexe polyT-polyA qui sert alors de point de départ à la réaction de transcription inverse réalisée par l'enzyme transcriptase inverse. On obtient alors différents ADN complémentaires des différents ARN messagers initialement présents dans l'échantillon sanguin ou dans les PBMCs. Dans la suite de l'exposé, on désigne par ADNc, un ADN complémentaire d'un ARN messager.

Par amorce d'amplification, on entend un fragment nucléotidique comprenant de 5 à 100 motifs nucléotidiques, préférentiellement de 15 à 25 motifs nucléotidiques et possédant une spécificité d'hybridation dans des conditions déterminées pour l'initiation d'une polymérisation enzymatique, par exemple dans une réaction d'amplification enzymatique.

Par réaction d'amplification enzymatique, on entend un processus générant de multiples copies d'un fragment nucléotidique cible à l'aide d'amorces d'amplification spécifiques par l'action d'au moins une enzyme. De telles réactions d'amplification sont bien connues de l'homme du métier et on peut citer notamment les techniques suivantes : PCR (Polymerase Chain Reaction), LCR (Ligase Chain Reaction), RCR (Repair Chain Reaction), 3SR (Self Sustained Séquence Replication) avec la demande de brevet WO-A-90/06995, NASBA (Nucleic Acid Sequence-Based Amplification), et TMA (Transcription Mediated Amplification) avec le brevet US-A-5,399,491.
On parle alors d'amplicons pour désigner les polynucléotides générés par une technique d'amplification enzymatique. Préférentiellement, lorsque l'amplification enzymatique est une PCR, le réactif spécifique comprend au moins 2 amorces d'amplification spécifiques afin d'amplifier une région particulière de l'ADN complémentaire de l'ARNm issu du gène cible. Lorsque l'amplification enzymatique est une PCR réalisée après une réaction de transcription inverse, on parle de RT-PCR.

Par sonde d'hybridation, on entend un fragment nucléotidique comprenant de 5 à 100 motifs nucléotidiques, notamment de 6 à 35 motifs nucléotidiques, possédant une spécificité d'hybridation dans des conditions déterminées pour former un complexe d'hybridation avec un fragment nucléotidique cible. Dans la présente invention, le fragment nucléotidique cible peut être une séquence nucléotidique comprise dans un ARN messager ou une séquence nucléotidique comprise dans un ADN complémentaire obtenu par transcription inverse dudit ARN messager.

Par hybridation, on entend le processus au cours duquel, dans des conditions appropriées, deux fragments nucléotidiques, tels que par exemple une sonde d'hybridation et un fragment nucléotidique cible, ayant des séquences suffisamment complémentaires sont susceptibles de former un double brin avec des liaisons hydrogènes stables et spécifiques. Un fragment nucléotidique "capable de s'hybrider" avec un polynucléotide est un fragment pouvant s'hybrider avec ledit polynucléotide dans des conditions d'hybridation, qui peuvent être déterminées dans chaque cas de façon connue. Les conditions d'hybridation sont déterminées par la stringence, c'est-à-dire la rigueur des conditions opératoires. L'hybridation est d'autant plus spécifique qu'elle est effectuée à plus forte stringence. La stringence est définie notamment en fonction de la composition en bases d'un duplex sonde/cible, ainsi que par le degré de mésappariement entre deux acides nucléiques. La stringence peut également être fonction des paramètres de la réaction, tels que la concentration et le type d'espèces ioniques présentes dans la solution d'hybridation, la nature et la concentration d'agents dénaturants et/ou la température d'hybridation. La stringence des conditions dans lesquelles une réaction d'hybridation doit être réalisée dépendra principalement des sondes d'hybridation utilisées. Toutes ces données sont bien connues et les conditions appropriées peuvent être déterminées par l'homme du métier. En général, selon la longueur des sondes d'hybridation utilisées, la température pour la réaction d'hybridation est comprise entre environ 20 et 70°C, en particulier entre 35 et 65°C dans une solution saline à une concentration d'environ 0,5 à 1 M. On réalise ensuite une étape de détection de la réaction d'hybridation.

Par détection on entend soit une détection directe par une méthode physique, soit une méthode de détection à l'aide d'un marqueur. De nombreuses méthodes de détection existent pour la détection des acides nucléiques [1,2].

Par marqueur, on entend un traceur capable d'engendrer un signal. Une liste non limitative de ces traceurs comprend les enzymes qui produisent un signal détectable par exemple par colorimétrie, fluorescence ou luminescence, comme la peroxydase de raifort, la phosphatase alcaline, la β-galactosidase, la glucose-6-phosphate déshydrogénase ; les chromophores comme les composés fluorescents, luminescents ou colorants ; les groupements à densité électronique détectables par microscopie électronique ou par leurs propriétés électriques comme la conductivité, par les méthodes d'ampérométrie ou de voltamétrie, ou par des mesures d'impédance ; les groupements détectables par des méthodes optiques comme la diffraction, la résonance plasmon de surface, la variation d'angle de contact ou par des méthodes physiques comme la spectroscopie de force atomique, l'effet tunnel, etc. ; les molécules radioactives comme ³²P, ³⁵S ou ¹²⁵I. Ainsi, le polynucléotide peut être marqué pendant l'étape d'amplification enzymatique, par exemple en utilisant un nucléotide triphosphate marqué pour la réaction d'amplification. Le nucléotide marqué sera un désoxyribonucléotide dans les systèmes d'amplification générant un ADN, comme la PCR, ou un ribonucléotide dans les techniques d'amplification générant un ARN, comme les techniques TMA ou NASBA. Le polynucléotide peut aussi être marqué après l'étape d'amplification, par exemple en hybridant une sonde marquée selon la technique d'hybridation sandwich décrite dans le document WO-A-91/19812.

Au sens de la présente invention, la sonde d'hybridation peut être une sonde dite de capture. Dans ce cas, le fragment nucléotidique cible peut être préalablement marqué au moyen d'un marqueur. La sonde dite de capture est immobilisée ou immobilisable sur un support solide par tout moyen approprié, c'est-à-dire directement ou indirectement, par exemple par covalence ou adsorption. On réalise alors une réaction d'hybridation entre ladite sonde de détection et le fragment nucléotidique cible marqué.

La sonde d'hybridation peut également être une sonde dite de détection. Dans ce cas, la sonde d'hybridation peut être marquée au moyen d'un marqueur. On réalise alors une réaction d'hybridation entre ladite sonde de capture et le fragment nucléotidique cible.

Que l'on utilise une sonde dite de capture ou une sonde dite de détection, la réaction d'hybridation peut être réalisée sur un support solide qui inclut tous les matériaux sur lesquels peut être immobilisé un acide nucléique. Des matériaux de synthèse ou des matériaux naturels, éventuellement modifiés chimiquement, peuvent être utilisés comme support solide, notamment les polysaccharides tels que les matériaux à base de cellulose, par exemple du papier, des dérivés de cellulose tels que l'acétate de cellulose et la nitrocellulose ou le dextrane, des polymères, des copolymères, notamment à base de monomères du type styrène, des fibres naturelles telles que le coton, et des fibres synthétiques telles que le nylon ; des matériaux minéraux tels que la silice, le quartz, des verres, des céramiques ; des latex ; des particules magnétiques ; des dérivés métalliques, des gels etc. Le support solide peut être sous la forme d'une plaque de microtitration, d'une membrane comme décrit dans la demande WO-A-94/12670, d'une particule ou d'une biopuce.

Dans la présente invention, la détermination de l'expression d'un gène cible peut être analysée par l'expression des ARNm qui sont transcrits à un temps donné. Dans ce cas, le matériel biologique est un acide nucléique, et le réactif spécifique peut être indifféremment une amorce d'amplification ou une sonde d'hybridation telles que définies précédemment.

On peut déterminer l'expression d'un gène cible de la manière suivante:
1) après avoir extrait les ARN totaux d'un échantillon sanguin ou de PBMCs, on réalise une étape de transcription inverse, telle que décrite précédemment afin d'obtenir les différents ADN complémentaires des différents ARN messagers initialement présents dans l'échantillon sanguin ou dans les PBMCs (ou ADNc)
2) on amplifie spécifiquement les ADNc. Dans ce cas, le réactif spécifique utilisé comprend au moins une amorce d'amplification spécifique du gène cible, telle que définie précédemment. Cette étape peut être réalisée notamment par une réaction d'amplification de type PCR ou par toute autre technique d'amplification appropriée
3) on détermine l'expression du gène cible en quantifiant les ADNc. Les ADNc peuvent être quantifiés notamment par l'utilisation d'une gamme de quantification obtenue par une réaction d'amplification conduite jusqu'à saturation. Afin de tenir compte de la variabilité d'efficacité enzymatique qui peut être observée lors des différentes étapes (transcription inverse, PCR quantitative...), on peut normaliser l'expression du gène cible des différents groupes de patients, par la détermination simultanée de l'expression d'un gène dit de référence dont l'expression est similaire chez les différents groupes de patients. En réalisant un rapport entre l'expression du gène cible et l'expression du gène de référence, on corrige ainsi toute variabilité entre les différentes expérimentations. L'homme du métier pourra se référer notamment aux publications suivantes : [3-4] Bustin SA Journal of molecular endocrinology, 2002, 29 : 23-39 ; Giulietti A Methods, 2001, 25 : 386-401

On peut également déterminer l'expression d'un gène cible de la manière suivante:
1) après avoir extrait les ARN totaux d'un échantillon sanguin ou des PBMCs, on réalise une étape de transcription inverse, telle que décrite précédemment afin d'obtenir les différents ADN complémentaires des différents ARN messagers initialement présents dans l'échantillon ou les PBMCs (ou ADNc)
2) on immobilise les ADNc sur une membrane
3) on détermine l'expression du gène cible en hybridant les ADNc à des sondes d'hybridation spécifiques du gène cible préalablement marquées. De telles techniques d'hybridation sont bien connues de l'homme du métier, et on peut citer notamment la technique du Northern blot. Cette réaction d'hybridation peut être réalisée après une étape d'amplification spécifique des ADN complémentaires des ARN messagers d'un gène cible lorsque notamment le gène est faiblement exprimé

L'expression d'un gène cible peut être également analysée par l'expression des protéines codées par le gène cible. Dans ce cas, le matériel biologique est une protéine ou un polypeptide et plusieurs techniques de détection avec ou sans ligand peuvent être utilisées. La spectrométrie de masse peut être utilisée comme technique de détection sans ligand. Le réactif spécifique peut être un ligand spécifique de la protéine codée par le gène cible pour un système de détection avec ligand.

En particulier, le ligand est un anticorps ou un dérivé d'anticorps (tel qu'un fragment d'anticorps) ou un analogue d'anticorps.

Les anticorps recombinants, spécifiques de la protéine traduite du gène cible peuvent être obtenus selon des procédés classiques connus de l'homme du métier, à partir d'organismes procaryotes, tels que bactéries, ou à partir d'organismes eucaryotes, tels que levures, cellules de mammifères, de plantes, d'insectes ou d'animaux, ou par des systèmes de production extra-cellulaire.

Les anticorps monoclonaux peuvent être préparés selon les techniques classiques connues de l'homme du métier telles que la technique des hybridomes dont le principe général est rappelé ci-dessous.

Dans un premier temps, on immunise un animal, généralement une souris, (ou des cellules en culture dans le cadre d'immunisations *in vitro*) avec un antigène cible d'intérêt, dont les lymphocytes B sont alors capables de produire des anticorps contre ledit antigène. Ces lymphocytes producteurs d'anticorps sont ensuite fusionnés avec des cellules myélomateuses "immortelles" (murines dans l'exemple) pour donner lieu à des hybridomes. A partir du mélange hétérogène des cellules ainsi obtenu, on effectue alors une sélection des cellules capables de produire un anticorps particulier et de se multiplier indéfiniment. Chaque hybridome est multiplié sous la forme de clone, chacun conduisant à la production d'un anticorps monoclonal dont les propriétés de reconnaissance vis-à-vis de l'antigène d'intérêt pourront être testées par exemple en ELISA, par immunotransfert en une ou deux dimensions, en immunofluorescence, ou à l'aide d'un biocapteur. Les anticorps monoclonaux ainsi sélectionnés, sont par la suite purifiés notamment selon la technique de chromatographie d'affinité.

Des fragments d'anticorps peuvent par exemple être obtenus par protéolyse. Ainsi, ils peuvent être obtenus par digestion enzymatique, résultant en des fragments de type Fab (traitement à la papaïne [5] ou de type F(ab)'2 (traitement à la pepsine; [6]. Ils peuvent également être préparés par voie recombinante [7]. Un autre fragment d'anticorps qui convient aux fins de l'invention comprend un fragment Fv qui est un dimère constitué de l'association non covalente du domaine variable léger (VL) et du domaine variable lourd (VH) du fragment Fab, donc de l'association de deux chaînes polypeptidiques. Afin d'améliorer la stabilité du fragment Fv due à la dissociation des deux chaînes polypeptidiques, ce fragment Fv peut être modifié par génie génétique en insérant un lien peptidique adapté entre le domaine VL et le domaine VH [8]. On parle alors de fragment scFv (« single chain Fragment variable ») car il est constitué d'une seule chaîne polypeptidique. L'utilisation d'un lien peptidique composé préférentiellement de 15 à 25 acides aminés permet de relier l'extrémité C-terminale d'un domaine à l'extrémité N-terminale de l'autre domaine, constituant ainsi une molécule monomérique dotée de propriétés de liaison similaires à celles de l'anticorps sous sa forme complète. Les deux orientations des domaines VL et VH conviennent (VL-lien-VH et VH-lien-VL) car elles présentent des propriétés fonctionnelles identiques. Bien entendu, tout fragment connu de l'homme du métier et présentant les caractéristiques immunologiques définies ci-dessus convient aux fins de l'invention.

Par analogue d'anticorps, on entend en particulier les protéines d'affinité aux propriétés compétitives (les nanofitines^{™}).

Lorsque le matériel biologique est un polypeptide ou une protéine issue de l'expression d'un gène, on peut déterminer l'expression de ce dernier, en détectant et ou quantifiant la dite protéine par Western Blot ou ELISA, ou toute autre méthode connue de l'homme du métier, telle qu'une méthode de chimiluminescence à partir du matériel biologique.

La technique ELISA (« Enzyme Linked Immuno Sorbent Assay ») est un dosage immunoenzymatique sur support solide. Ce test entre dans le cadre plus général des EIA (« Enzyme Immunoassays ») dans lequel le dosage est couplé à une réaction catalysée par une enzyme. La technique utilise un ou deux anticorps. L'anticorps de détection de la formation de complexes immuns (antigène / anticorps) est couplé à l'enzyme et peut générer l'émission d'un signal par un substrat chromogène ou fluorogène.

La technique Western Blot est un test pour détecter une protéine spécifique dans un échantillon à l'aide d'un anticorps spécifique de cette protéine comprenant les étapes suivantes telle que décrites ci-dessous.

La première étape est une électrophorèse sur gel, qui permet de séparer les protéines de l'échantillon selon leur taille.

Les protéines dans le gel sont alors transférées sur une membrane (nitrocellulose, PVDF...) par pression ou par application d'un courant électrique, les protéines se fixant à la membrane grâce à des interactions hydrophobes et ioniques.
Après saturation des sites d'interaction non spécifique, un premier anticorps, spécifique de la protéine à étudier (anticorps primaire) est incubé avec la membrane.
La membrane est ensuite rincée afin d'enlever les anticorps primaires non liés, puis incubée avec des anticorps dits secondaires, qui vont se lier aux anticorps primaires. Cet anticorps secondaire est habituellement lié à une enzyme qui permet l'identification visuelle de la protéine étudiée sur la membrane. L'ajout d'un substrat marqué de l'enzyme engendre une réaction colorée qui est visible sur la membrane.

### FIGURES

Figure 1 : Les figures 1A et 1B représentent respectivement les niveaux d'expression de LILRB2 avec 2 probesets Affymetrix indépendants : 207967_x_at et 210146_x_at. Les données sont exprimées en intensité de fluorescence pour les 3 groupes de patients : volontaires sains (VS), patients non survivants (NS) et survivants (S).
Figure 2 : La figure 2 représente la corrélation de l'intensité de fluorescence pour LILRB2 avec les 2 probesets Affymetrix.
Figure 3 : La figure 3 montre les niveaux d'expression de LILRB1 avec le probeset Affymetrix 211336_x_at. Les données sont exprimées en intensité de fluorescence pour les 3 groupes de patients : volontaires sains (VS), patients non survivants (NS) et survivants (S).
Figure 4 : La figure 4 représente la corrélation de l'intensité de fluorescence entre les 2 probesets Affymetrix des gènes LILRB1 (211336_x_at) et LILRB2 (207697_x_at).
Figure 5: La figure 5 montre la corrélation entre l'intensité de fluorescence du probeset (207697_x_at) obtenue avec la plateforme Affymetrix et les mesures CNRQ de LILRB2 obtenues en RT-qPCR sur la cohorte de découverte.
Figure 6: La figure 6 présente les CNRQ de LILRB2 pour les patients survivants (S) et non survivants (NS).
Figure 7 : La figure 7 représente la courbe ROC de l'association entre les valeurs de CNRQ de LILRB2 et la probabilité de décès chez les patients en choc septique.
Figure 8 : La figure 8 représente la courbe de survie de Kaplan Meier pour les patients présentant des valeurs de CNRQ pour LILRB2 au-dessus de la médiane (0,86) en caractères gras et en dessous de la médiane en caractères normaux.

### EXEMPLES: Recherche d'un profil d'expression pour le pronostic de survie ou de sévérité d'un patient en choc septique

### Exemple 1 : Description des patients

### 1.1 Cohorte de découverte

L'étude a été réalisée sur une cohorte de 51 patients en choc septique âgés de 25 à 85 ans (33 hommes, 18 femmes, âge médian: 66 ans) et admis dans le service de réanimation du centre hospitalier Lyon Sud - Lyon (France) entre 2004 et 2009 pour la prise en charge d'un choc septique. Leurs caractéristiques sont décrites dans le tableau 1 ci-dessous. Tous les patients étaient en état de choc septique nécessitant un traitement par les aminés après remplissage vasculaire. Le début du traitement aux catécholamines est considéré comme le T0 du choc septique et de l'étude. Parmi les 51 patients de la cohorte, 17 patients (33%) sont décédés dans les 28 premiers jours après mise sous traitement par catécholamine (patients non survivants : NS) et 34 patients ont survécu au-delà de 28 jours (patients survivants : S). Les patients âgés de moins de 18 ans sont exclus de l'étude.

La sévérité de l'affection a été calculée par le score SAPS-II (Simplified Acute Physiology Score) établi par Le Gall, et al. en 1993 qui prend en compte différents paramètres cliniques (l'âge, la fréquence cardiaque, la température centrale, la PaO2/FiO2, la diurèse, l'urée sanguine, les globules blancs, la kaliémie, la natrémie, la bicarbonatémie, la bilirubine, le type d'admission, les pathologies associées et le score de Glasgow) et également le score de SOFA (Sequential Organ Failure Assessment score) qui prend en compte les défaillances d'organes (basé sur six notes différentes, une pour chacune des voies respiratoires, cardio-vasculaires, hépatiques, coagulation, les systèmes rénaux et neurologiques). Ces scores sont inversement proportionnels à la survie. Pour le groupe de patients étudié, le SAPSII médian est 51 et le SOFA médian est 10. Toutes ces informations sont résumées dans le tableau 1 ci-dessous.

**Tableau 1**

| | | **Non survivants n= 17 (33%)** | **Survivants n= 34 (67%)** | **Total n=51 (%)** | **P-value** |
|---|---|---|---|---|---|
| Femme n (%) | | 9 (53) | 9 (26) | 18 (35) | 0.062 |
| Homme n (%) | | 8 (47) | 25 (74) | 33 (65) | |
| Age (années) médiane (Q1-Q3) | | 59 (48-70) | 66 (56-76) | 66 (54-74) | 0.418 |
| SAPS II médiane (Q1-Q3) | | 64 (56-70) | 47 (38-55) | 51 (43-61) | <.001 |
| SOFA médiane (Q1-Q3) | | 12 (10-15) | 9 (8-11) | 10 (8-12) | 0.002 |
| Type d'infection | communautaire n(%) | 8 (47) | 17 (50) | 25 (49) | ns |
| | nosocomiale n (%) | 8 (47) | 17 (50) | 25 (49) | |
| Site d' infection | abdominale n (%) | 4 (38) | 15 (40) | 19 (39) | 0.291 |
| | pulmonaire n (%) | 10 (46) | 13 (40) | 23 (42) | |
| | autres n (%) | 4 (16) | 6 (20) | 10 (19) | |

### 1.2 Série de contrôle

Des sujets volontaires sains (VS) ont été recrutés afin d'obtenir une série contrôle dont les échantillons ont été traités et analysés dans des conditions identiques à celles des malades. Il s'agit de 24 sujets sains (12 femmes et 12 hommes) d'un âge médian de 57 ans. Ces informations sont résumées dans le tableau 2 ci-dessous.

**Tableau 2**

| | **VS** |
|---|---|
| | **n=24(%)** |
| Femme n (%) | 12 (50) |
| Homme n (%) | 12 (50) |
| Age (années) médiane (Q1-Q3) | 57 52-60) |

### 1.3 Cohorte de Validation :

La cohorte de validation inclut 262 patients en choc septique de 19 à 93 ans (164 hommes, 93 femmes, âge médian: 67 ans) admis dans 6 services de réanimation de Lyon (France) entre 2009 et 2011 pour la prise en charge d'un choc septique. Ces patients sont tous en état de choc nécessitant un traitement par les aminés après remplissage vasculaire. Le début du traitement aux catécholamines est considéré comme le T0 du choc septique. Parmi les 262 inclus dans l'étude, 90 patients (34%) sont décédés dans les 28 premiers jours après mise sous traitement par catécholamine.

Les patients âgés de moins de 18 ans sont exclus de l'étude.

La sévérité de l'affection est calculée par le score SAPS-II et par le score de SOFA. Pour le groupe de patients étudié le SAPS-II médian est 62 et le SOFA médian est 12.
Le tableau 3 ci-dessous résume les informations sur les patients de la cohorte de validation.

**Tableau 3**

| | | **Non survivants n= 90 (34%)** | **Survivants n= 172 (66%)** | **Total n=262 (%)** | **P-value** |
|---|---|---|---|---|---|
| Femme n (%) | | 33 (36.7) | 65 (37.8) | 98 (37.4) | 0.965 |
| Homme n (%) | | 57 (63.3) | 107 (62.2) | 164 (62.6) | |
| Age (années) | médiane (Q1-Q3) | 71.5 (63-78) | 65 (56-77) | 67 (58-78) | 0.003 |
| SAPS II médiane (Q1-Q3) | | 77 (63-93) | 57 (44-68) | 62 (50-77) | <.001 |
| SOFA médiane (Q1-Q3) | | 14(12-16) | 11 (9-13) | 12 (10-14) | <.001 |
| Type d'infection | communautaire n (%) | 48 (53.3) | 114 (66.3) | 162 (61.8) | 0.041 |
| | nosocomiale n (%) | 42 (46.7) | 58 (33.7) | 100 (38.2) | |
| Site d' infection | abdominale n (%) | 30 (33.3) | 41 (23.8) | 71 (27.1) | 0.212 |
| | pulmonaire n (%) | 36 (40.0) | 72 (41.9) | 108 (41.2) | |
| | autres n (%) | 24 (26.7) | 59 (34.3) | 83 (31.7) | |

### Exemple 2 : Echantillonnage et collection des données :

Les premiers échantillons sanguins sont obtenus au plus tard 12 heures après le début du choc septique.

### 2.1 Extraction des ARN totaux de l'échantillon biologique:

Les prélèvements ont été collectés directement dans des tubes PAXgene^{™} Blood RNA (PreAnalytix, Hilden, Allemagne). Après prélèvement, les tubes ont été laissés à température ambiante pendant 4 heures puis conservés à -80°C jusqu'à l'extraction du matériel biologique dans le but d'optimiser et de standardiser les conditions de conservation des échantillons. Plus précisément, dans ce protocole, les ARN totaux ont été extraits à l'aide des kits PAXgene^{™} Blood RNA® (PreAnalytix) en respectant les recommandations du fabriquant.

La qualité des ARN totaux extraits a été analysée par le bio analyseur AGILENT 2100 (Agilent Technologies, Waldbronn, Germany) à l'aide du système Lab-on-chip RNA 6000 Nano Assay (Agilent). Les ARN totaux comprennent les ARN de transfert, les ARN messagers (ARNm) et les ARN ribosomaux.

### 2.2 Etude de découverte :

Pour l'étude de découverte, la réaction de transcription inverse des ARNm est réalisée selon le protocole d'Affymetrix à partir de 50 ng d'ARN total. Une amorce oligonucléotidique a été utilisée pour cibler tous les ARN pendant la transcription inverse. L'ensemble des étapes a été réalisé avec le kit WTO NuGen et hybridé sur des puces à ADN. L'hybridation de l'ARNc est réalisée avec les biopuces GeneChipHuman Genome U133 Plus 2 avant l'étape d'amplification du signal par l'incubation avec le mélange SAPE contenant la Streptavidine Phycoerythrine puis les anticorps IgG de chèvre anti Streptavidine mélangés avec l'anticorps biotinylé anti IgG de chèvre. Cette dernière étape utilise la plateforme Fluidic FS450. L'analyse des données Affymetrix débute par la capture de l'image de la biopuce par le scanner GeneChip® 3000 d'Affymetrix. Les données sont ensuite normalisées par RMA (Robust Multiple-Array Average). Toutes les données basées sur l'intensité du signal sont utilisées après la normalisation. Les différences de niveaux d'expression en ARNm entre les patients décédés dans les 28 premiers jours (patients non survivants) et les patients survivants sont déterminées par la méthode SAM (Tusher, Tibshirani, and Chu, 2001).

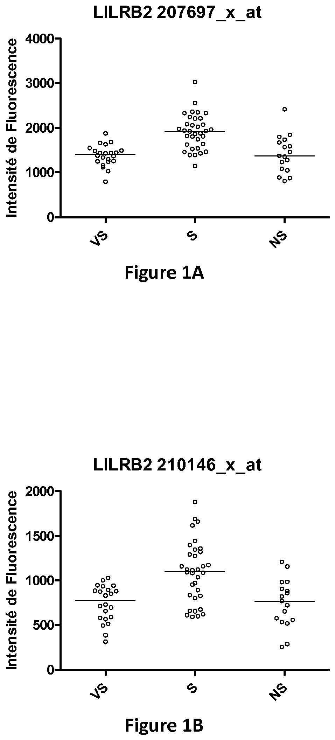

Parmi les gènes différentiellement exprimés entre les patients survivants, non survivants et les contrôles sains, on constate une surexpression d'LILRB2 chez les patients survivants comparée au niveau d'expression obtenue à la fois chez les contrôles sains et chez les non survivants dont les niveaux d'expression sont comparables. Ces résultats sont présentés à la figure 1 annexée avec 2 probesets Affymetrix indépendants, respectivement identifiés en 207697_x_at et 210146_x_at, ce qui confirme la robustesse et la fiabilité des résultats. Dans la figure 1, respectivement figure 1A et figure 1B, les données sont présentées en intensité de fluorescence pour les 2 probesets Affymetrix 207697_x_at et 210146_x_at, respectivement, pour les 3 groupes de patients : volontaires sains (cf. tableau 2), patients non survivants et survivants (cf. tableau 1).

L'intensité de fluorescence des probesets 207697_x_at et 210146_x_at a été comparée pour les patients survivants et non survivants. Les résultats sont présentés ci-dessous.

| Probeset | **SFC** | **AUC** | **p-value** |
|---|---|---|---|
| 207697_x_at | 1,53 | 0,84 | 0,0002 |
| 210146_x_at | 1,19 | 0,78 | 0,0012 |

| | | | |
|---|---|---|---|
| **SFC:** Standard Fold Change **AUC:** Area Under Curve **p-value:** selon le test de Mann Whitney | | | |

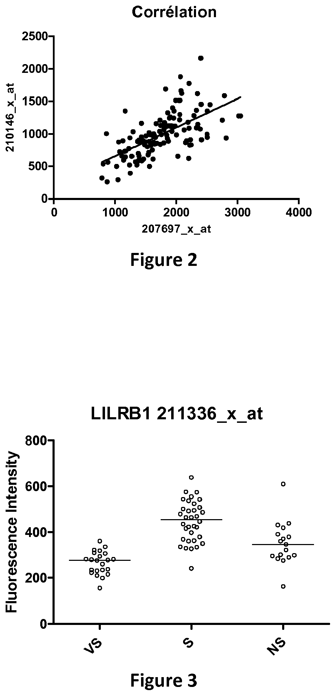

L'intensité de fluorescence a ensuite été corrélée pour les 2 probsets Affymetrix. Les résultats sont présentés à la figure 2.

| | |
|---|---|
| Spearman coefficient corrélation r | 0, 68 |
| Intervalle de confiance 95% | 0,565 to 0,762 |
| Spearman test signivicativité P value | < 0,0001 |

Comme cela ressort de la figure 2, on constate une très bonne corrélation pour les 2 probesets Affymetrix.

Parmi les gènes différentiellement exprimés entre les patients survivants, non survivants et les contrôles sains, on constate aussi une surexpression d'LILRB1 chez les patients survivants comparée au niveau d'expression obtenue à la fois chez les contrôles sains et chez les non survivants. Ces résultats sont présentés à la figure 3 annexée avec 1 probeset Affymetrix indépendants, respectivement identifiés en 207697_x_at et 211336_x_at pour les 3 groupes de patients : volontaires sains, patients non survivants et survivants. Les résultats sont données en intensité de fluorescence.

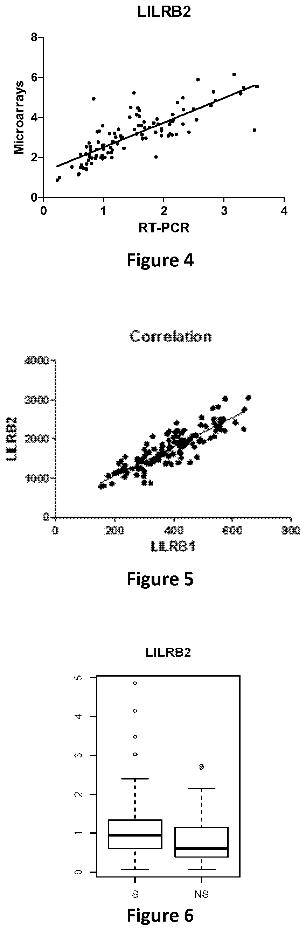

L'intensité de fluorescence entre les 2 probesets Affymetrix des gènes LILRB1 (211336_x_at) et LILRB2 (207697_x_at) a ensuite été corrélée. Les résultats sont présentés à la figure 4. On observe une corrélation significative (0.86[0.81-0.90]) entre les résultats d'expression des deux gènes LILRB2 et LILRB1.

### 2.3 Validation technique (transfert de plateforme) :

Afin de confirmer les résultats à l'aide d'une autre technique de biologie moléculaire, l'expression d'ARN de LILRB2 a été mesurée par RT-PCR quantitative sur la même cohorte.
Brièvement, une réaction de transcription reverse (RT) a été réalisée à partir de 200 ng d'ARN total dans un volume final de 20 µl à l'aide des kits SuperScript® VILO^{™} system (Invitrogen, Carlsbad, CA, USA) en respectant les recommandations du fabriquant. Chaque solution de cDNA a été diluée au 1/10 dans de l'eau DEPC. Un standard a été préparé pour le gène d'intérêt (LILRB2) et le gène de référence (HPRT1) par une amplification PCR (polymerase chain reaction) conduite jusqu'à saturation. Les amplicons obtenus ont été purifiés (PCR purification kit, Qiagen Ltd) et la présence d'un amplicon unique a été vérifié par électrophorèse capillaire à l'aide du bioanalyseur AGILENT 2100 (Agilent Technologies, Waldbronn, Germany) et du système Lab-on-chip DNA Assay (Agilent).
Le LightCycler^{™} 480 (Roche) a été utilisé et les réactions PCR ont été effectuées à l'aide du kit LightCycler® 480 Probes Master (Roche Molecular Biochemicals). Chaque PCR a été effectuée dans un volume final de 20 µl contenant 10 µl de LightCycler® 480 Probes Master, 2x conc, 2 µl de amorces-sonde MIX, 10x conc contenant les amorces sens et anti-sens (0.5 µM concentration finale) ainsi que la sonde Taqman (0.1µM concentration finale), et 8 µl de solution de cDNA dilué au 20ème. Après une étape de dénaturation de 10 min à 95°C, l'amplification a été effectuée à l'aide de 40 cycles d'un protocole de "touch-down" PCR (10 sec à 95°C, 29 sec d'hybridation à 68-58°C, suivi d'une extension de 11 sec à 72°C). A la fin de chaque cycle, la fluorescence libérée par la sonde d'hydrolyse Taqman a été mesurée.

Les combinaisons d'amorces nécessaires à la quantification du gène de référence HPRT1 et du gène d'intérêt LILRB2 sont décrites ci-dessous. Pour HPRT1, le n° d'accession Genbank est NM_000194.2 et la région amplifiée est 630-788. Pour LILRB2, variant 1, le n° d'accession Genbank est NM_005874.3 et la région amplifiée est 2006-2182 et pour le variant 2, le numéro d'accession Genebank est NM_001080978.2 et la région amplifiée est 1791-1879.
LILRB2 :

| | | |
|---|---|---|
| Amorce sens 5'-->3' | CCAGAGCCCACAGACAGAGG | (SEQ ID NO :1) |
| Amorce anti-sens 5'-->3' | TGTCCTTCACGGCAGCATAGA | (SEQ ID NO :2) |
| Sonde TaqMan | GACTCCACACTCAGTAGAAG | (SEQ ID NO :3) |

HPRT1 : amplicon 158pb

| | | |
|---|---|---|
| Amorce sens 5'-->3' | CCAAAGATGGTCAAGGTCGC | (SEQ ID NO :4) |
| Amorce anti-sens 5'-->3' | GACACAAACATGATTCAAATCC | (SEQ ID NO :5) |
| Sonde TaqMan | CAAGTTTGTTGTAGGATATGCCC | (SEQ ID NO :6) |

La quantité d'ARNm cible LILRB2 relative à la quantité d'ARNm du gène de référence HPRT1 a été analysée par la technique de quantification relative avec le LightCycler Relative Quantification Software (Roche Molecular Biochemicals). La "Second Derivative Maximum Method" du logiciel LightCyclerTM (Roche) a été utilisée pour déterminer automatiquement le Crossing Point (Cp) pour chaque échantillon. La valeur du Cp a été définie comme le nombre de cycles pour lequel la fluorescence était significativement différente du bruit de fond.
Cinq dilutions en séries au 1/10 ont été réalisées en quadruplicate avec chaque standard afin de générer une courbe d'étalonnage exprimant le Cp en fonction du logarithme du nombre de copies. Les dilutions de standard ont été optimisées afin que la courbe d'étalonnage couvre le niveau d'expression attendu pour le gène cible et le gène de référence. Les courbes standards relatives décrivant l'efficacité de PCR pour le gène cible et le gène de référence ont été générées et utilisées pour réaliser une quantification relative appelée CRNQ (calibrated normalized relative quantities) décrite par Jan Hellemans et *al.* (qBase relative quantification framework and software for management and automated analysis of real-time quantitative PCR data. Genome Biology 2007)
La figure 5 annexée montre la corrélation entre l'intensité de fluorescence du probeset (207697_x_at) obtenue avec la plateforme Affymetrix et les mesures CNRQ obtenues en RT-qPCR sur la cohorte de découverte pour le gène LILRB2. Les résultats montrent une corrélation significative (0.81[0.72-0.86]) entre les résultats Affymetrix et ceux de RT-PCR quantitative, confirmant la pertinence du gène.

| | |
|---|---|
| Spearman coefficient corrélation r | 0,8075 |
| Intervalle de confiance 95% | 0,7254 to 0,8670 |
| Spearman test significativité P value | < 0,0001 |

### 2.9 Validation biologique:

Afin de valider l'association entre l'expression de LILRB2 et la prédiction de survie chez les patients en choc septique, l'expression des ARNm de LILRB2 a été mesurée par RT-PCR quantitative sur une cohorte de validation (incluant 262 patients en choc septique, avec 172 survivants et 90 non survivants (décédés dans les 28 premiers jours) (cf. tableau 3)

La figure 6 présente les CNRQ de LILRB2 pour les patients survivants (S) et non survivants (NS).

Un modèle logistique a été utilisé pour décrire l'association entre LILRB2 et la probabilité de décès. Le modèle logistique décrit la probabilité de mourir avant le 28^{ème} jour. La mesure d'association entre la probabilité de décès et la covariable est l'Odds Ratio (OR). Cette mesure d'association peut être interprétée comme un risque relatif. Par exemple, un OR = 2 pour le groupe 1 versus le groupe 2 signifie que la probabilité de décès pour les patients du groupe 1 est égal à 2 fois la probabilité de mourir pour les patients du groupe 2.

L'Odds Ratio pour un incrément de 1 point du CNRQ de LILRB2 est 0.47 (p=0.0028). L'Odds Ratio entre les patients présentant des valeurs de CNRQ pour LILRB2 = 1.3 (3ème quartile) et 0.5 (1^{er} quartile) est 0.55. Ainsi, les patients avec un niveau d'expression de LILRB2 élevé ont une probabilité de décès significativement plus faible de décéder que les patients avec un niveau d'expression plus faible.

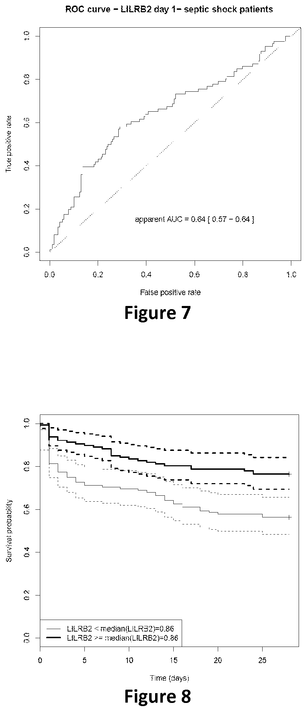

La figure 7 représente la courbe ROC de l'association entre les valeurs de LILRB2 et la probabilité de décès chez les patients en choc septique.

LILRB2 est également associé à la date de survie des patients en choc septique (et non seulement à la probabilité de décès dans les 28 premiers jours). La figure 8 représente la courbe de survie de Kaplan Meier pour les patients en choc septique à J1 présentant des valeurs de CNRQ pour LILRB2 au-dessus de la médiane (0,86) caractères gras et en dessous de la médiane en caractères normaux.

### REFERENCES BIBLIOGRAPHIQUES

1. Thomas Shanley et al. Molecular Medicine, vol. 13, no. 9-10
2. Hector R. Wang et al. Critical Care Medicine, vol. 37, no. 5
3. Hector R. Wang. Critical Care, vol. 16

## Revendications

1. Procédé pour établir *in vitro* un pronostic de sévérité chez un patient en choc septique, comprenant les étapes suivantes:
(i) à partir d'un échantillon biologique provenant dudit patient, on mesure *in vitro* le niveau d'expression du produit d'expression d'au moins un gène choisi parmi les gènes *lilrb2* et *lilrb1*, (ii) on compare le niveau d'expression du produit d'expression dudit au moins un gène avec un niveau d'expression contrôle de bon pronostic de sévérité pour le produit d'expression du même gène, dans lequel un niveau d'expression du produit d'expression dudit au moins un gène en-dessous dudit niveau d'expression contrôle est indicatif d'un mauvais pronostic de sévérité pour le patient.

2. Procédé selon la revendication 1, dans lequel
(i) on mesure le niveau d'expression du produit d'expression du gène *lilrb2*, (ii) on compare le niveau d'expression du produit d'expression du gène *lilrb2* avec le niveau d'expression contrôle du gène *lilrb2,*
dans lequel le niveau d'expression du produit d'expression du gène *lilrb2* du patient au-dessous du niveau d'expression contrôle est indicatif d'un mauvais pronostic pour le patient.

3. Procédé selon la revendication 1, dans lequel
(i) on mesure le niveau d'expression du produit d'expression du gène *lilrb2* et le niveau d'expression du produit d'expression du gène *lilrb1* du patient, (ii) on compare le niveau d'expression du produit d'expression du gène *lilrb2* et du gène *lilrb1* du patient respectivement avec le niveau d'expression contrôle de chaque gène,
dans lequel un niveau d'expression du produit d'expression du gène *lilr2* et du gène *lilrb1* du patient au-dessous respectivement du niveau d'expression contrôle seuil prédéterminé de chaque gène est indicatif d'un mauvais pronostic pour le patient.

4. Procédé selon la revendication 1, dans lequel ledit échantillon biologique est choisi parmi le sang, le plasma, le sérum, la salive, l'urine, le liquide céphalo-rachidien, le liquide pleural et le liquide péritonéal.

5. Procédé selon la revendication 1, dans lequel le produit d'expression du gène est un ARN, de préférence un ARN messager ou un ADNc.

6. Procédé selon la revendication 1, dans lequel le produit d'expression est une protéine ou un polypeptide.

7. Procédé selon la revendication 5, dans lequel le niveau d'expression de l'ARN est déterminé à l'aide d'au moins une sonde d'hybridation spécifique du produit d'expression.

8. Procédé selon la revendication 7, dans lequel le niveau d'expression de l'ARN est déterminé par une amplification enzymatique quantitative.

9. Procédé selon la revendication 6, dans lequel le niveau d'expression du polypeptide est déterminé par un dosage immunologique quantitatif.

10. Procédé pour réaliser *in vitro* un suivi d'un patient en choc septique, par mesure de l'évolution du niveau d'expression du produit d'expression d'au moins un gène choisi parmi les gènes *lilrb2* et *lilrb1* chez ledit patient, dans lequel
(i) on mesure le niveau d'expression dudit au moins un gène dans un échantillon du patient prélevé à J1,
(ii) on mesure le niveau d'expression dudit au moins un gène dans un échantillon du patient prélevé à Jx,
dans lequel une augmentation dudit niveau d'expression dudit au moins un gène entre J1 et Jx indique que le patient évolue vers un bon pronostic de sévérité.

11. Procédé selon la revendication 10, dans lequel ledit niveau d'expression est mesuré par amplification quantitative.

12. Procédé selon la revendication 10, dans lequel le niveau d"expression est mesuré par un dosage immunologique quantitatif.

## Patentansprüche

1. Verfahren zur *in vitro*-Stellung einer Prognose der Schwere bei einem Patienten mit septischem Schock, das die folgenden Schritte umfasst:
(i) man misst ausgehend von einer von dem Patienten entnommenen biologischen Probe *in vitro* den Expressionsspiegel des Expressionsprodukts von mindestens einem Gen, das aus den Genen lilrb2 und lilrb1 ausgewählt ist, (ii) man vergleicht den Expressionsspiegel des Expressionsprodukts des mindestens einen Gens mit einem Kontrollexpressionsspiegel des Expressionsprodukts desselben Gens mit einer guten Prognose der Schwere, wobei ein Expressionsspiegel des Expressionsprodukts des mindestens einen Gens unterhalb des Kontrollexpressionsspiegels auf eine schlechte Prognose der Schwere für den Patienten hindeutet.

2. Verfahren nach Anspruch 1, wobei
(i) man den Expressionsspiegel des Expressionsprodukts des lilrb2-Gens misst, (ii) man den Expressionsspiegel des Expressionsprodukts des lilrb2-Gens mit dem Kontrollexpressionsspiegel des lilrb2-Gen vergleicht,
wobei ein Expressionsspiegel des Expressionsprodukts des lilrb2-Gens des Patienten unterhalb des Kontrollexpressionsspiegels auf eine schlechte Prognose für den Patienten hindeutet.

3. Verfahren nach Anspruch 1, wobei
(i) man den Expressionsspiegel des Expressionsprodukts des lilrb2-Gens und den Expressionsspiegel des Expressionsprodukts des lilrb1-Gens des Patienten misst, (ii) man den Expressionsspiegel des Expressionsprodukts des lilrb2-Gens und des lilrb1-Gens des Patienten mit dem jeweiligen Kontrollexpressionsspiegel jedes Gens vergleicht,
wobei ein Expressionsniveau des Expressionsprodukts des lilrb2-Gens und des lilrb1-Gens des Patienten jeweils unterhalb des zuvor bestimmten Schwellen-Kontrollexpressionsspiegels des jeweiligen Gens auf eine schlechte Prognose für den Patienten hindeutet.

4. Verfahren nach Anspruch 1, wobei die biologische Probe aus Blut, Plasma, Serum, Speichel, Urin, Zerebrospinalflüssigkeit, Pleuraflüssigkeit und Peritonealflüssigkeit ausgewählt ist.

5. Verfahren nach Anspruch 1, wobei das Expressionsprödukt des Gens eine RNA, vorzugsweise eine Boten-mRNA, oder eine cDNA ist.

6. Verfahren nach Anspruch 1, wobei das Expressionsprodukt ein Protein oder ein Polypeptid ist.

7. Verfahren nach Anspruch 5, wobei der Expressionsspiegel der RNA mithilfe mindestens einer für das Expressionsprodukt spezifischen Hybridisierungssonde bestimmt wird.

8. Verfahren nach Anspruch 7, wobei der Expressionsspiegel der RNA durch eine quantitative enzymatische Amplifikation bestimmt wird.

9. Verfahren nach Anspruch 6, wobei der Expressionsspiegel des Polypeptids durch eine quantitative immunologische Bestimmung bestimmt wird.

10. Verfahren zur Durchführung einer Überwachung eines Patienten mit septischem Schock *in vitro* durch Messen der Veränderung des Expressionsspiegels des Expressionsprodukts von mindestens einem Gen, das aus den Genen lilrb2 und lilrb1 ausgewählt ist, bei dem Patienten, wobei
(i) man den Expressionsspiegel des mindestens einen Gens in einer an T1 von dem Patienten entnommenen Probe misst,
(ii) man den Expressionsspiegel des mindestens einen Gens in einer an Tx von dem Patienten entnommenen Probe misst,
wobei eine Erhöhung des Expressionsspiegels des mindestens einen Gens zwischen T1 und Tx darauf hindeutet, dass sich der Patient zu einer guten Prognose der Schwere hin verändert.

11. Verfahren nach Anspruch 10, wobei der Expressionsspiegel durch quantitative Amplifikation gemessen wird.

12. Verfahren nach Anspruch 10, wobei der Expressionsspiegel durch eine quantitative immunologische Bestimmung gemessen wird.

## Claims

1. Method for making an *in vitro* prognosis of severity for a patient in septic shock, comprising the following steps:
(i) the level of expression of the expression product of at least one gene chosen from the *lilrb2* and *lilrb1* genes is measured in *vitro* on the basis of a biological sample taken from said patient,
(ii) the level of expression of the expression product of said at least one gene is compared with a control level of expression, of the expression product of the same gene, with a good prognosis of severity, in which a level of expression of the expression product of said at least one gene below said control level of expression indicates a poor prognosis of severity for the patient.

2. Method according to Claim 1, in which
(i) the level of expression of the expression product of the *lilrb2* gene is measured, (ii) the level of expression of the expression product of the *lilrb2* gene is compared with the control level of expression of the *lilrb2* gene,
in which a level of expression of the expression product of the *lilrb2* gene in the patient below the control level of expression indicates a poor prognosis for the patient.

3. Method according to Claim 1, in which
(i) the patient's level of expression of the expression product of the *lilrb2* gene and the patient's level of expression of the expression product of the *lilrb1* gene are measured, (ii) the patient's level of expression of the expression product of the *lilrb2* gene and of the *lilrb1* gene are compared respectively with the control level of expression of each gene,
in which a level of expression of the expression product of the *lilrb2* gene and *of* the *lilrb1* gene in the patient respectively below the predetermined threshold control level of expression of each gene indicates a poor prognosis for the patient.

4. Method according to Claim 1, in which said biological sample is chosen from blood, plasma, serum, saliva, urine, cerebrospinal fluid, pleural fluid and peritoneal fluid.

5. Method according to Claim 1, in which the expression product of the gene is an RNA, preferably a messenger RNA or a cDNA.

6. Method according to Claim 1, in which the expression product is a protein or a polypeptide.

7. Method according to Claim 5, in which the level of expression of the RNA is determined by means of at least one hybridization probe specific for the expression product.

8. Method according to Claim 7, in which the level of expression of the RNA is determined by quantitative enzymatic amplification.

9. Method according to Claim 6, in which the level of expression of the polypeptide is determined by a quantitative immunoassay.

10. Method for carrying out *in vitro* monitoring of a patient in septic shock, by measuring the change in the level of expression of the expression product of at least one gene chosen from the *lilrb2* and *lilrb1* genes in said patient, in which
(i) the level of expression of said at least one gene in a sample taken from the patient on D1 is measured,
(ii) the level of expression of said at least one gene in a sample taken from the patient on Dx is measured,
in which an increase in said level of expression of said at least one gene between D1 and Dx indicates that the patient is moving towards a good prognosis of severity.

11. Method according to Claim 10, in which said level of expression is measured by quantitative amplification.

12. Method according to Claim 10, in which the level of expression is measured by a quantitative immunoassay.
